# EUROPEAN PATENT SPECIFICATION

(11) **EP 1 014 955 B1**
(45) Date of publication and mention of the grant of the patent: **07.04.2004**
(21) Application number: 98901619.1
(22) Date of filing: 13.01.1998
(51) Int. Cl.: A61K 9/72, A61K 31/35

(54) **NEW FORMULATION FOR INHALATION HAVING A POURED BULK DENSITY OF FROM 0.28 TO 0.38 G/ML, COMPRISING TERBUTALINE SULPHATE, A PROCESS FOR PREPARING THE FORMULATION AND THE USE THEREOF**
NEUARTIGE FORMULIERUNG ZUR INHALATION MIT EINER BULKDICHTE ZWISCHEN 0.28 UND 0.38 G/ML, MIT TERBUTALINSULPHAT, VERFAHREN ZUR HERSTELLUNG DER FORMULIERUNG SOWIE ANWENDUNG DERSELBEN
NOUVELLE FORMULATION POUR INHALATION AYANT UNE DENSITE EN VRAC DE 0,28 A 0,38 G/ML ET CONTENANT DU SULFATE DE TERBUTALINE, PROCEDE DE PREPARATION ET UTILISATION DE CETTE FORMULATION

(30) Priority: 20.01.1997 SE 9700136
(43) Date of publication of application: 05.07.2000
(73) Proprietor: AstraZeneca AB, 151 85 Södertälje (SE)
(72) Inventor: TROFAST, Jan, S-226 47 Lund (SE)
(86) International application number: PCT/SE1998/000041
(87) International publication number: WO 1998/031353

(56) References cited:
- US-A- 4 590 206
- US-A- 5 551 489

## Description

### Field of the Invention

The present invention provides a new pharmaceutical formulation, its preparation and its use.

### Background to the Invention

Potent drugs for administration by inhalation are generally formulated in association with carriers such as lactose because of the problem of preparing accurate doses. When such drugs are diluted, variations in the weight of the formulation result in a smaller drug dosage variation rate compared with when they are not diluted. These formulations have generally consisted of coarse particles of the carrier with fine particles of the drug, which combination is generally known as an ordered mixture.

US 4 590 206 discloses a finely divided inhalation drug comprising a therapeutically effective proportion of individual particles capable of penetrating deep into the lung, characterised in that a bulk of the particles which is both unagglomerated and unmixed with a coarse carrier, is sufficiently free flowing to be filled into capsules on an automatic filing machine and to empty from an opened capsule in an inhalation device. It is preferred that the particles have a loose bulk density of greater than about 0.3 glcm³ and that they contain medicament and water only. However, where a highly active medicament is used, diluent may be incorporated into the particles.

US 5 551 489 discloses a method of treating a finely divided powdered medicament where the medicament may be selected from terbutaline, budesonide and lactose. The agglomerates of the powder typically have a bulk density of from about 0.2 to 0.4 glml.

The invention provides an improved formulation which, in systems designed to imitate inhalation has been found to give an improved dispersion of the drug.

### Description of the Invention

According to the invention there is provided a dry powder composition comprising terbutaline sulphate and a carrier substance, both of which have a mass median diameter of less than 10µm and are substantially uniformly distributed wherein the formulation has a poured bulk density of from 0.28 to 0.38 g/ml.

The poured bulk density according to the present invention is measured using known techniques, for example those described in "Powder testing guide: Methods of measuring the physical properties of Bulk powders" L. Svarovsky, Elsevier Applied Science 1987, pp 84-86.

The carrier substance is preferably a mono-, di- or polysaccharide, a sugar alcohol or another polyol. Suitable carriers are, for example, lactose, glucose, raffinose, melezitose, lactitol, maltitol, trehalose, sucrose, mannitol; and starch. Lactose is particularly preferred, especially in the form of its monohydrate.

The ingredients of the formulation according to the invention must both be in a finely divided form, i.e. their mass median diameter is less than 10 µm, preferably from 1 to 7 µm, as measured by a laser diffraction instrument or a coulter counter. The ingredients may be produced in the desired particle size using methods known to those of skill in the art, e.g. milling, micronisation or direct precipitation.

The composition according to the invention is preferably formulated to comprise, as a daily dose, from 50 µg to 8 mg, more preferably from 100 µg to 4 mg and most preferably from 125 µg to 2 mg of terbutaline sulphate. More preferably the composition is formulated to provide unit doses of 125, 250 or 500 µg of terbutaline sulphate. The composition is preferably formulated to comprise in each unit dose from 50 µg to 25 mg of the carrier substance, more preferably from 50 µg to 10mg, most preferably from 100 to 4000 µg.

According to the invention there is further provided a process for preparing a composition according to the invention which comprises
(a) micronising terbutaline sulphate and the carrier substance;
(b) optionally conditioning the product; and
(c) spheronizing until the desired bulk density is obtained.

The process preferably further comprises a low energy remicronisation step after step (b).

The formulation according to the invention may be made by conventional techniques known *per se*. Such production processes generally comprise micronising the ingredients to the required size, removing any amorphous areas on the particles obtained by, for example, the methods described in WO 92/18110 or WO 95/05805 and then agglomerating, spheronising and sieving the powder obtained. The size of the agglomerates obtained is preferably in the range of from 100 to 2000 µm, more preferably from 100 to 800 µm. The bulk density of the formulation produced may be adjusted by varying the components and the process empirically, for example the bulk density can be increased by lengthening the time in which the particles are tumbled in a spheronising device.

In solid-solid mixing, one of the most important features is to ensure content uniformity. The major problem encountered in the powder mixing of fine powders is the inability of mixers to break down powder agglomerates. It has been found that a remicronisation step after the conditioning step of the fine powder with low energy input is advantageous. It should generally be carried out using enough energy to break down powder agglomerates but not with so much energy that the size of the particles themselves is affected. Such a step gives a composition wherein the active substance and carrier substance are substantially uniformly distributed, having for example a relative standard deviation of less than 3% (preferably less than 1%) and does not disturb the crystallinity of the fine particles.

The formulation according to the invention may be administered using any known dry powder inhaler, for example the inhaler may be a single or a multi dose inhaler, and may be a breath actuated dry powder inhaler, for example Turbuhaler (trade mark). The invention further provides use of a composition according to the invention in the manufacture of a medicament for use in therapy. The composition according to the invention is useful in the treatment of respiratory disorders, particularly asthma. The invention also provides a method of treating a patient suffering from a respiratory disorder which comprises administering to the patient a therapeutically effective amount of a composition according to the invention.

The invention is illustrated, but not limited, by reference to the following Examples.

### Example 1

60 Parts of terbutaline sulphate were micronized to a mass medium diameter of less than 2 µm in a Alpin mill 100AFG and thereafter conditioned according to the method described in US 5562923. 40 Parts of lactose monohydrate were micronized (Alpin mill 100AFG) down to a mass medium diameter of less than 3 µm and thereafter conditioned according to the method described in WO 95/05805. The micronized and conditioned terbutaline sulphate and lactose monohydrate were mixed thoroughly in a Turbula mixer. The mixture was remicronised in a spiral jet mill at a pressure of only about 1 bar to obtain an evenly distributed mixture. The powder was then agglomerated by feeding the powder into a twin screw feeder (K-Tron), sieving in an oscillating sieve (0.5 mm mesh size), spheronising in a rotating pan with a peripheral speed of 0.5m/s for 4 minutes and then sieving again using the same sieve, then spheronising once more for 6 minutes before final sieving (mesh size 1.0 mm) giving a powder with a bulk density of 0.28 g/ml.

### Example 2

Example 1 was repeated with 30 parts of terbutaline sulphate and 70 parts of lactose monohydrate to give a powder with a bulk density of 0.31 g/ml.

## Claims

1. A dry powder composition comprising terbutaline sulphate and a carrier substance, both of which have a mass median diameter of less than 10 µm and are substantially uniformly distributed, wherein the composition has a poured bulk density of from 0.28 to 0.38 g/ml.

2. A composition according to claim 1 wherein the bulk density is from 0.30 to 0.36 g/ml.

3. A composition according to claim 1 or 2 wherein the carrier substance is selected from lactose, glucose, raffinose, melezitose, lactitol, maltitol, trehalose, sucrose, mannitol and starch.

4. A composition according to claim 3 wherein the carrier substance is lactose monohydrate.

5. A composition according to any one of claims 1 to 4 for use in the treatment of a respiratory disorder.

6. A process for preparing a composition according to claim 1 which comprises
(a) micronising terbutaline sulphate and the carrier substance;
(b) optionally conditioning the product; and
(c) spheronizing until the desired bulk density is obtained.

7. A process according to claim 6 which comprises a low energy remicronisation step after step (b).

8. Use of a composition according to any one of claims 1 to 4 in the manufacture of a medicament for use in therapy.

9. Use of a composition according to any one of claims 1 to 4 in the manufacture of a medicament for use in respiratory disorders.

## Patentansprüche

1. Eine Trockenpulverzusammensetzung, enthaltend Terbutalinsulfat und eine Trägersubstanz, beide jeweils mit einem massenmittleren Durchmesser von weniger als 10 µm und im wesentlichen gleichmäßig verteilt, wobei die Zusammensetzung eine Schüttdichte von 0,28 bis 0,38 g/ml aufweist.

2. Zusammensetzung nach Anspruch 1, wobei die Schüttdichte von 0,30 bis 0,36 g/ml beträgt.

3. Zusammensetzung nach einem der Ansprüche 1 oder 2, wobei die Trägersubstanz aus Lactose, Glucose, Raffinose, Melezitose, Lactit, Maltit, Trehalose, Saccharose, Mannit und Stärke ausgewählt ist.

4. Zusammensetzung nach Anspruch 3, wobei es sich bei der Trägersubstanz um Lactose-monohydrat handelt.

5. Zusammensetzung nach einem der Ansprüche 1 bis 4 zur Verwendung bei der Behandlung einer Atemwegserkrankung.

6. Verfahren zur Herstellung einer Zusammensetzung nach Anspruch 1, bei dem man
(a) Terbutalinsulfat und Trägersubstanz mikronisiert;
(b) das Produkt gegebenenfalls konditioniert und
(c) sphäronisiert, bis die gewünschte Schüttdichte erreicht ist.

7. Verfahren nach Anspruch 6, bei dem sich an Schritt (b) ein Remikronisierungsschritt bei niedriger Energie anschließt.

8. Verwendung einer Zusammensetzung nach einem der Ansprüche 1 bis 4 zur Herstellung eines Medikaments zur Verwendung in der Therapie.

9. Verwendung einer Zusammensetzung nach einem der Ansprüche 1 bis 4 zur Herstellung eines Medikaments zur Verwendung bei Atemwegserkrankungen.

## Revendications

1. Composition pulvérulente sèche comprenant du sulfate de terbutaline et une substance véhicule, tous les deux présentant un diamètre moyen en masse inférieur à 10 µm et étant distribués substantiellement uniformément, où la composition présente une densité en vrac au versage de 0,28 à 0,38 g/ml.

2. Composition selon la revendication 1, dans laquelle la densité en vrac est de 0,30 à 0,36 g/ml.

3. Composition selon la revendication 1 ou 2, dans laquelle la substance véhicule est choisie parmi le lactose, le glucose, le raffinose, le mélézitose, le lactitol, le maltitol, le tréhalose, le sucrose, le mannitol et l'amidon.

4. Composition selon la revendication 3, dans laquelle la substance véhicule est le monohydrate de lactose.

5. Composition selon l'une quelconque des revendications 1 à 4, destinée à être utilisée pour le traitement d'un trouble respiratoire.

6. Procédé de préparation d'une composition selon la revendication 1, comprenant
(a) la micronisation du sulfate de terbutaline et de la substance véhicule ;
(b) le conditionnement éventuel du produit ; et
(c) la sphéronisation jusqu'à l'obtention de la densité en vrac souhaitée.

7. Procédé selon la revendication 6, comprenant une étape de remicronisation à faible énergie après l'étape (b).

8. Utilisation d'une composition selon l'une quelconque des revendications 1 à 4, pour la fabrication d'un médicament destiné à être utilisé en thérapie.

9. Utilisation d'une composition selon l'une quelconque des revendications 1 à 4, pour la fabrication d'un médicament destiné à être utilisé pour les troubles respiratoires.
